# EUROPÄISCHE PATENTSCHRIFT

(11) **EP 0 846 692 B1**
(45) Veröffentlichungstag und Bekanntmachung des Hinweises auf die Patenterteilung: **04.10.2001**
(21) Anmeldenummer: 97120717.0
(22) Anmeldetag: 26.11.1997
(51) Int. Cl.: C07D 275/04

(54) **Verfahren zur Herstellung von 3-Chlorbenzisothiazolen**
Process for preparation of 3-chlorobenzisothiazoles
Procédé pour la préparation de 3-chlorobenzisothiazoles

(30) Priorität: 09.12.1996 DE 19651038
(43) Veröffentlichungstag der Anmeldung: 10.06.1998
(73) Patentinhaber: BAYER AG, 51368 Leverkusen (DE)
(72) Erfinder: Kraus, Helmut, Dr., 51519 Odenthal (DE); Krebs, Andreas, Dr., 51519 Odenthal (DE); Söllner, Robert, Dr., 51373 Leverkusen (DE)

(56) Entgegenhaltungen:
- EP-A- 0 196 096
- DE-A- 2 029 387
- DE-A- 3 530 089
- H. BÖSHAGEN ET AL.: CHEMISCHE BERICHTE, Bd. 101, 1968, Seiten 2472-84, XP002059323
- P. V. PLAZZI ET AL.: ATENEO PARMENSE, ACTA NAT., Bd. 15, 1979, Seiten 49-55, XP002059324
- M. H. NORMAN ET AL.: JOURNAL OF LABELLED COMPOUNDS AND RADIOPHARMACEUTICALS, Bd. 38, Nr. 3, März 1996, Seiten 269-80, XP002059325
- J. FAUST ET AL.: ZEITSCHRIFT FÜR NATURFORSCHUNG, Bd. 20b, Nr. 7, 1965, Seite 712 XP002059326
- J. FAUST: ZEITSCHRIFT FÜR CHEMIE, Bd. 8, 1968, Seiten 170-1, XP002059327

## Beschreibung

Die vorliegende Erfindung betrifft ein Verfahren zur Herstellung von 3-Chlorbenzisothiazolen durch Umsetzung der entsprechenden Benzisothiazolone mit Phosgen in Gegenwart eines Katalysators.

3-Chlorbenzisothiazole sind wichtige Zwischenprodukte zur Herstellung von pharmazeutischen Wirkstoffen (siehe z.B. US-A 5 206 366 und EP-A1 281 309) und Pflanzenschutzmitteln (siehe z.B. DE-A1 20 29 387).

Die Herstellung von 3-Chlorbenzisothiazolen ist beispielsweise ausgehend von Diphenyldisulfiddicarbonsäuren-(2,2') über die Amide zu 3-Chlor-1,2-benzisothiazoliumchloriden und anschließende Pyrolyse möglich (siehe EP-A1 196 096).

Üblicherweise geht man jedoch von den z.T. handelsüblichen Benzisothiazolonen aus und chloriert diese mit Phosphoroxychlorid (siehe z.B. DE-A1 35 30 089 und Chem. Ber. 101, 2472 (1968)). Dabei werden in 60 bis 80 %iger Ausbeute die 3-Chlorbenzisothiazole erhalten. Allerdings muß man zur Abtrennung der aus dem Phosphoroxychlorid entstehenden Polyphosphorsäuren wäßrig aufarbeiten. Dadurch werden Extraktionen mit zwei organischen Lösungsmitteln, eine Aktivkohle-Klärung und eine Destillation nötig. Wegen der zu entsorgenden phosphorhaltigen Abwässer und der aufwendigen Aufarbeitung ist dieses Verfahren aufwendig und nicht wirtschaftlich.

Die Umsetzung von N-substituierten Benzisothiazolen mit Oxalylchlorid und mit Phosgen zu 3-Chlor-1,2-benzisothiazoliumchloriden ist in 90 %iger Ausbeute beschrieben (siehe Z. Naturforschung 20b, 712 (1965) und Z. Chem 8, 170 (1968)). Wenn man dieses Verfahren auf N-unsubstituierte Benzisothiazole überträgt, stellt man fest, daß lange Reaktionszeiten benötigt werden und sich die Ausbeuten drastisch vermindern (siehe das vorliegende Vergleichsbeispiel).

Es wurde nun ein Verfahren zur Herstellung von N-unsubstituierten 3-Chlorbenzisothiazolen der Formel 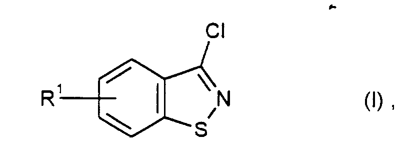 in der
- R¹: für Wasserstoff, Halogen, Nitro, C₁-C₆-Alkyl oder C₁-C₆-Alkoxy steht,
gefunden, das dadurch gekennzeichnet ist, daß man N-unsubstituierte Benzisothiazolone der Formel 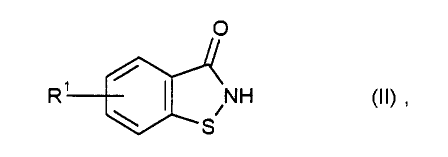 in der
- R¹: die bei Formel (I) angegebene Bedeutung hat,
mit Phosgen in Gegenwart von Katalysatoren der Formel (III) umsetzt 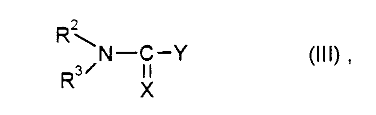 in der
- R² und R³: unabhängig voneinander jeweils für C₁-C₆-Alkyl oder C₅-C₇-Cycloalkyl,
- X: für Sauerstoff oder NH und
- Y: für Wasserstoff oder einen Rest der Formel 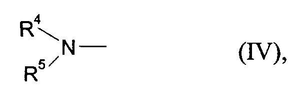 stehen, in der
- R⁴ und R⁵: unabhängig voneinander jeweils für C₁-C₆-Alkyl oder C₅-C₇-Cycloalkyl stehen,
wobei im Falle X = NH Y nur für einen Rest der Formel (IV) steht und
im Falle X = Sauerstoff und Y = einem Rest der Formel (IV)
R⁴ und R⁵ auch gemeinsam für
a) -(CHR⁶)ₙ- mit n = einer ganzen Zahl von 2 bis 4,
b) -CHR⁶-O-CHR⁶- oder
c) 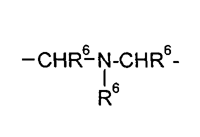
mit R⁶ unabhängig voneinander jeweils = Wasserstoff, C₁-C₆-Alkyl oder C₅-C₇-Cycloalkyl stehen können,
wobei man das Verfahren in Gegenwart eines Verdünnungsmittels bei Reaktionstemperaturen von 50 bis 150°C durchführt, den Katalysator der Formel (III) in einer Menge einsetzt, die 0,5 bis 200 Mol-%, bezogen auf das eingesetzte Benzisothiazolon der Formel (II), entspricht, pro Mol Benzisothiazolon der Formel (II) 1,2 bis 10 Mol Phosgen einsetzt und man nach Beendigung der Phosgenaufnahme noch einige Zeit bei Reaktionstemperatur oder einer anderen Temperatur im Bereich von 50 bis 150°C nachrührt, dann überschüssiges Phosgen entfernt, anschließend bei Normaldruck oder im schwachen Vakuum gegebenenfalls das Verdünnungsmittel entfernt und das Reaktionsprodukt durch Destillation im Vakuum gewinnt.
Halogen steht beispielsweise für Fluor, Chlor oder Brom.

C₁-C₆-Alkyl steht jeweils beispielsweise für Methyl, Ethyl, n-Propyl, i-Propyl, n-Butyl, i-Butyl, sec.-Butyl, tert.-Butyl, Amyl oder Hexyl. Bevorzugt sind Methyl, Ethyl, n-Propyl, i-Propyl, n-Butyl, i-Butyl, sec.-Butyl, tert.-Butyl.

C₁-C₆-Alkoxy kann beispielsweise und vorzugsweise einen Alkylteil enthalten, wie er unmittelbar oben bei den C₁-C₆-Alkylresten näher erläutert ist.

C₅-C₇-Cycloalkyl steht beispielsweise für Cyclopentyl, Cyclohexyl oder Cycloheptyl.

Bevorzugte Benzisothiazolone der Formel (II) sind unsubstituiertes Benzthiazolon und N-unsubstituierte Benzthiazolone, bei denen R¹ für Chlor steht.

Bevorzugte Katalysatoren der Formel (III) sind solche, bei denen
a) X für Sauerstoff und Y für Wasserstoff stehen und R² und R³ gleich sind und jeweils für C₁-C₄-Alkyl stehen,
b) X für Sauerstoff oder NH und Y für einen Rest der Formel (IV) stehen, wobei R² bis R⁵ gleich sind und jeweils für Methyl oder Ethyl stehen und
c) X für Sauerstoff und Y für einen Rest der Formel (IV) stehen, wobei R² und R³ gleich sind und für C₁-C₄-Alkyl stehen und R⁴ und R⁵ gemeinsam für -(CH₂)₂- oder -(CH₂)₃- stehen.

Bevorzugt werden erfindungsgemäß 3-Chlorbenzisothiazole der Formel (I) hergestellt, bei denen R¹ für Wasserstoff oder Chlor steht.

Vorzugsweise führt man das erfindungsgemäße Verfahren wegen des hohen Schmelzpunktes der Benzisothiazolone der Formel (II) in Verdünnungsmitteln durch. In Frage kommen z.B. Toluol, Xylole, Isopropylbenzole, Chlorbenzol, Dichlorbenzole, Diphenyl, Methylcyclohexan und höhere aliphatische Kohlenwasserstoffe wie beispielsweise Octan, Nonan und Dekan. Man kann auch Gemische dieser Lösungsmittel einsetzen.

Wenn man nur Benzisothiazolone der Formel (II) zur Verfügung hat, die noch Wasser enthalten, so ist es zweckmäßig, vor der Durchführung des erfindungsgemäßen Verfahrens dieses Wasser zu entfernen, beispielsweise durch azeotrope Destillation, wobei als Hilfsmittel für die azeotrope Destillation vorzugsweise das für die Reaktion vorgesehene Verdünnungsmittel verwendet wird, beispielsweise eine chlorierte aromatische Verbindung.

Bevorzugt liegt die Reaktionstemperatur bei 80 bis 130°C.

Der Katalysator der Formel (III) wird in einer Menge von 0,5 bis 200 Mol-%, bezogen auf das eingesetzte Benzisothiazolon der Formel (II) eingesetzt. Bevorzugt beträgt diese Menge 0,7 bis 100 Mol-%, besonders bevorzugt 1 bis 20 Mol-%.

Pro Mol Benzisothiazolon der Formel (II) setzt man 1,2 bis 10 Mol Phosgen ein. Vorzugsweise liegt diese Menge bei 1,3 bis 5 Mol, insbesondere bei 1,4 bis 3 Mol. Man kann auch so verfahren, dass man so lange Phosgen einleitet, bis vom Reaktionsgemisch keines mehr aufgenommen wird.

Vorzugsweise führt man das erfindungsgemäße Verfahren so durch, dass man das Edukt, den Katalysator und gegebenenfalls das Verdünnungsmittel vorlegt und bei der gewünschten Reaktionstemperatur Phosgen einleitet.

Überschüssiges Phosgen und sonstige entweichende Reaktionsgase können über einen mit Wasser berieselten Aktivkohleturm abgeleitet werden. Bevorzugt kondensiert man jedoch einen Großteil des entweichenden Phosgens in einem Tieftemperaturkühler bei z.B. -15 bis -75°C. Man kann dann dieses Phosgen, gegebenenfalls nach einer entsprechenden Reinigung, erneut in Phosgenierungsreaktionen einsetzen, beispielsweise auch in das erfindungsgemäße Verfahren.

Zur Aufarbeitung des Reaktionsgemisches entfernt man zunächst gegebenenfalls noch vorhandenes Phosgen, beispielsweise durch Hindurchleiten von Stickstoff. Dann entfernt man bei Normaldruck oder schwachem Vakuum das Lösungsmittel und gewinnt das Reaktionsprodukt durch Destillation im Vakuum. So kann man i.a. über 98 % reines 3-Chlorbenzisothiazol der Formel (I) in Ausbeuten von bis über 90 % der Theorie (nach der Destillation) erhalten.

Die erfindungsgemäß bei der Herstellung von N-unsubstituierten Chlorbenzisothiazolen erzielbaren Vorteile sind außerordentlich überraschend, da diese Reaktion nicht analog zu entsprechenden Reaktionen zur Herstellung von N-substituierten Chlorbenzisothiazolen verläuft und die bekannten Verfahren zur Herstellung von N-substituierten Chlorbenzisothiazolen nicht auf die Herstellung von N-unsubstituierten Chlorbenzthiazolen übertragen werden können.

### Beispiele

### Beispiel 1

565 g Benzisothiazolon (Gehalt: 92,8 Gew.-%; flüchtige Bestandteile: 28,05 Gew.-%; insgesamt effektiver Gehalt: 66,8 Gew.-% = 2,5 mol) wurden mit 500 ml Chlorbenzol versetzt und vorhandenes Wasser azeotrop ausgeschleust. Dabei wurden etwa 150 ml Wasser abgeschieden und destilliertes Chlorbenzol wieder rückgeführt.

Nach der Zugabe von 20,2 g Tetramethylguanidin wurden bei 120°C 400 g Phosgen eingeleitet. Anschließend wurde für 2 Stunden Stickstoff durch das Reaktionsgemisch geleitet und danach bei 150°C Sumpftemperatur das Lösungsmittel bei Normaldruck abdestilliert. Nach einer Vakuumdestillation (130°C/10 mbar) wurden 373 98 % reines 3-Chlorbenzisothiazol (= 86,3 % der Theorie) erhalten.

### Beispiel 2

406 g trockenes, 93 % reines Benzisothiazolon wurden analog Beispiel 1 mit 20,3 g Tetramethylharnstoff in o-Chlorbenzol phosgeniert. Die Vakuumdestillation ergab 3-Chlorbenzisothiazol in einer Ausbeute von 84,9 % der Theorie.

### Beispiel 3

Es wurde verfahren wie in Beispiel 2, jedoch wurden als Katalysator 28 g Dibutylformamid und als Lösungsmittel 600 ml Nonan verwendet. Es wurde 3-Chlorbenzisothiazol in einer Ausbeute von 78,8 % der Theorie erhalten.

### Beispiel 4

190 g trockenes 97,7 gew.-%ig reines Benzisothiazolon wurden in 500 ml Chlorbenzol vorgelegt. Danach wurden 5,1 g 1,3-Dimethyltetrahydro-2-(1H)-pyrimidinon zugefügt, das Reaktionsgemisch auf 120°C erwärmt und innerhalb von 5 Stunden etwa 250 g Phosgen eingeleitet. Die den Reaktor verlassenden Gase wurden in einem Kühler weitgehend kondensiert, der mit einem Kältemittel von -25°C betrieben wurde.

Nach der Entphosgenierung des Reaktionsgemisches durch 2-stündiges Durchleiten von Stickstoff wurde zunächst bei Normaldruck Chlorbenzol abdestilliert und anschließend im Vakuum 3-Chlorbenzisothiazol. Die Ausbeute betrug 85,6 % der Theorie.

### Beispiel 5

Es wurde analog Beispiel 4 gearbeitet, jedoch wurden als Katalysator 4,5 g 1,3-Dimethylimidazolidinon verwendet. Nach der Vakuumdestillation wurde 3-Chlorbenzisothiazol in einer Ausbeute von 89,3 % der Theorie erhalten.

### Beispiel 6

Es wurde analog Beispiel 5 gearbeitet wobei 4 kg 93 gew.-%ig reines Benzisothiazolon und 90 g 1,3-Dimethylimidazolidinon in 51 Chlorbenzol phosgeniert wurden. Zur Gewinnung des Reaktionsproduktes wurden vor der Vakuumdestillation 200 ml hochsiedendes Paraffinöl zugesetzt und eine 20 cm lange Vigreux-Kolonne verwendet. Man erhielt 3-Chlorbenzisothiazol in einer Reinheit von 99,4 Gew.-% und in einer Ausbeute von 92,7 % der Theorie.

### Beispiel 7 (zum Vergleich)

### (Analog Z. Naturforsch. 20b, 712 (1965) und Z. Chem. 8, 170 (1968))

190 g trockenes, 97,7 gew.-%ig reines Benzisothiazolon wurden in 500 ml Xylol bei 130°C ohne Zusatz eines Katalysators 24 Stunden lang phosgeniert. Die Untersuchung des dann vorliegenden Reaktionsgemisches durch HPLC zeigte, daß die Lösung 3-Chlorbenzisothiazol in einer Menge enthielt, die einer Ausbeute von 16,3% der Theorie entsprach. Die Lösung enthielt außerdem 38,8 Gew.-% des ursprünglich eingesetzten Benzisothiazols.

## Patentansprüche

1. Verfahren zur Herstellung von N-unsubstituierten 3-Chlorbenzisothiazolen der Formel 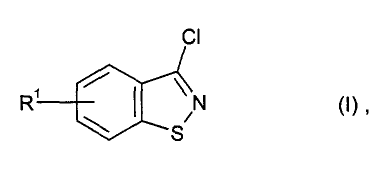 in der
R¹ für Wasserstoff, Halogen, Nitro, C₁-C₆-Alkyl oder C₁-C₆-Alkoxy steht,
**dadurch gekennzeichnet, dass** man N-unsubstituierte Benzisothiazolone der Formel 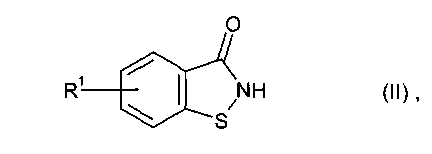 in der
R¹ die bei Formel (I) angegebene Bedeutung hat,
mit Phosgen in Gegenwart von Katalysatoren der Formel (III) umsetzt 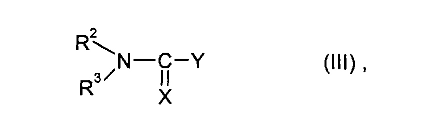 in der
R² und R³ unabhängig voneinander jeweils für C₁-C₆-Alkyl oder C₅-C₇-Cycloalkyl,
x für Sauerstoff oder NH und
Y für Wasserstoff oder einen Rest der Formel 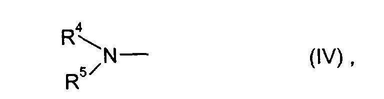 stehen, in der
R⁴ und R⁵ unabhängig voneinander jeweils für C₁-C₆-Alkyl oder C₅-C₇-Cycloalkyl stehen,
wobei im Falle X = NH Y nur für einen Rest der Formel (IV) steht
und
im Falle X = Sauerstoff und Y = einem Rest der Formel (IV)
R⁴ und R⁵ auch gemeinsam für
a) -(CHR⁶)ₙ- mit n = einer ganzen Zahl von 2 bis 4,
b) -CHR⁶-O-CHR⁶- oder
c) 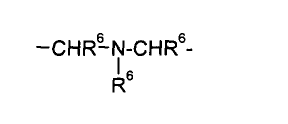 mit R⁶ unabhängig voneinander jeweils = Wasserstoff, C₁-C₆-Alkyl oder C₅-C₇-Cycloalkyl stehen können,
wobei man das Verfahren in Gegenwart eines Verdünnungsmittels bei Reaktionstemperaturen von 50 bis 150°C durchführt, den Katalysator der Formel (III) in einer Menge einsetzt, die 0,5 bis 200 Mol-%, bezogen auf das eingesetzte Benzisothiazolon der Formel (II), entspricht, pro Mol Benzisothiazolon der Formel (II) 1,2 bis 10 Mol Phosgen einsetzt und man nach Beendigung der Phosgenaufnahme noch einige Zeit bei Reaktionstemperatur oder einer anderen Temperatur im Bereich von 50 bis 150°C nachrührt, dann überschüssiges Phosgen entfernt, anschließend bei Normaldruck oder im schwachen Vakuum gegebenenfalls das Verdünnungsmittel entfernt und das Reaktionsprodukt durch Destillation im Vakuum gewinnt.

2. Verfahren nach Anspruch 1, **dadurch gekennzeichnet, dass** in den Formeln Halogen für Fluor, Chlor oder Brom,
C₁-C₆-Alkyl jeweils für Methyl, Ethyl, n-Propyl, i-Propyl, n-Butyl, i-Butyl, sec.-Butyl, tert.-Butyl, Amyl oder Hexyl,
C₁-C₆-Alkoxy für Methoxy, Ethoxy, n-Propoxy, i-Propoxy, n-Butoxy, i-Butoxy, sec.-Butoxy, tert.-Butoxy, Amyloxy oder Hexyloxy und
C₅-C₇-Cycloalkyl für Cyclopentyl, Cyclohexyl oder Cycloheptyl
stehen.

3. Verfahren nach Ansprüchen 1 und 2, **dadurch gekennzeichnet, dass** man Benzisothiazolone der Formel (II) einsetzt, bei denen R¹ für Wasserstoff oder Chlor steht.

4. Verfahren nach Ansprüchen 1 bis 3, **dadurch gekennzeichnet, dass** als Katalysatoren der Formel (III) solche eingesetzt werden, bei denen
a) X für Sauerstoff und Y für Wasserstoff stehen und R² und R³ gleich sind und jeweils für C₁-C₄-Alkyl stehen,
b) X für Sauerstoff oder NH und Y für einen Rest der Formel (IV) stehen, wobei R² bis R⁵ gleich sind und jeweils für Methyl oder Ethyl stehen oder
c) X für Sauerstoff und Y für einen Rest der Formel (IV) stehen, wobei R² und R³ gleich sind und für C₁-C₄-Alkyl stehen und R⁴ und R⁵ gemeinsam für -(CH₂)₂- oder -(CH₂)₃- stehen.

5. Verfahren nach Ansprüchen 1 bis 4, **dadurch gekennzeichnet, dass** man das Edukt, den Katalysator und gegebenenfalls das Verdünnungsmittel vorlegt und bei der gewünschten Reaktionstemperatur Phosgen einleitet.

## Claims

1. Process for preparing N-unsubstituted 3-chlorobenzisothiazoles of the formula 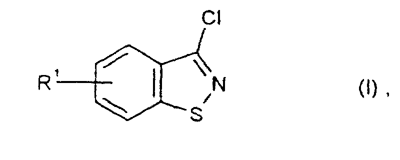 in which
R¹ represents hydrogen, halogen, nitro, C₁-C₆-alkyl or C₁-C₆-alkoxy,
**characterized in that** N-unsubstituted benzisothiazolones of the formula 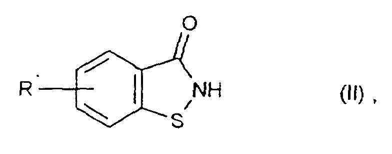 in which
R¹ is as defined for formula (I), are reacted with phosgene in the presence of catalysts of the formula (III) 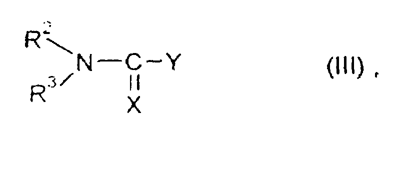
in which
R² and R³ independently of one another each represent C₁-C₆-alkyl or C₅-C₇-cycloalkyl,
X represents oxygen or NH and
Y represents hydrogen or a radical of the formula 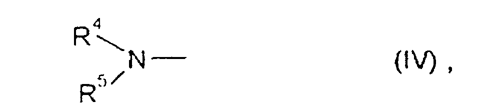 in which
R⁴ and R⁵ independently of one another each represent C₁-C₆-alkyl or C₅-C₇-cycloalkyl,
where Y in the case of X = NH only represents a radical of the formula (IV) and
where R⁴ and R⁵ together in the case of X = oxygen and Y = a radical of the formula (IV) also represent
a) -(CHR⁶)ₙ- where n = an integer from 2 to 4,
b) -CHR⁶-O-CHR⁶- or
c) 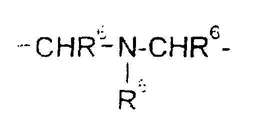 where each R⁶ independently of the others may represent hydrogen, C₁-C₆-alkyl or C₅-C₇-cycloalkyl,
where the process is carried out in the presence of a diluent at reaction temperatures of from 50 to 150°C, the catalyst of the formula (III) is employed in an amount corresponding to 0.5 to 200 mol%, based on the benzisothiazolone of the formula (II) employed, 1.2 to 10 mol of phosgene are employed per mole of benzisothiazolone of the formula (II), and the reaction mixture is stirred for some time at the reaction temperature or any other temperature in the range from 50 to 150°C after the phosgene uptake has ended, excess phosgene is then removed, the diluent, if appropriate, is subsequently removed under atmospheric pressure or under slightly reduced pressure and the reaction product is obtained by vacuum distillation.

2. Process according to Claim 1, **characterized in that** in the formulae halogen represents fluorine, chlorine or bromine,
C₁-C₆-alkyl represents methyl, ethyl, n-propyl, i-propyl, n-butyl, i-butyl, sec-butyl, tert-butyl, amyl or hexyl,
C₁-C₆-alkoxy represents methoxy, ethoxy, n-propoxy, i-propoxy, n-butoxy, i-butoxy, sec-butoxy, tert-butoxy, amyloxy or hexyloxy and
C₅-C₇-cycloalkyl represents cyclopentyl, cyclohexyl or cycloheptyl.

3. Process according to Claims 1 and 2, **characterized in that** benzisothiazolones of the formula (II) are employed in which R¹ represents hydrogen or chlorine.

4. Process according to Claims 1 to 3, **characterized in that** those catalysts of the formula (III) are employed in which
a) X represents oxygen and Y represents hydrogen and R² and R³ are identical and each represents C₁-C₄-alkyl,
b) X represents oxygen or NH and Y represents a radical of the formula (IV) where R² to R⁵ are identical and each represents methyl or ethyl or
c) X represents oxygen and Y represents a radical of the formula (IV) where R² and R³ are identical and each represents C₁-C₄-alkyl and R⁴ and R⁵ together represent -(CH₂)₂- or -(CH₂)₃-.

5. Process according to Claims 1 to 4, **characterized in that** the starting material, the catalyst and, if appropriate, the diluent are initially charged and phosgene is introduced at the desired reaction temperature.

## Revendications

1. Procédé pour la préparation de 3-chlorobenzoisothiazoles non substitués à l'azote et répondant à la formule 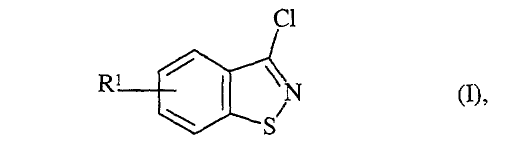 dans laquelle
R¹ représente l'hydrogène, un halogène, un groupe nitro, alkyle en C₁-C₆ ou alcoxy en C₁-C₆,
**caractérisé en ce que** l'on fait réagir des benzoisothiazolones non substituées à l'azote et répondant à la formule 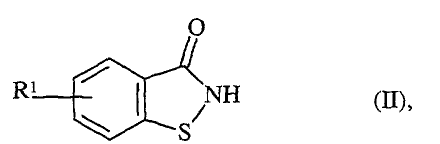 dans laquelle
R¹ a les significations indiquées en référence à la formule (I), avec le phosgène en présence de catalyseurs de formule (III) 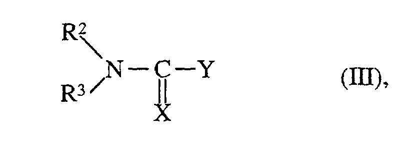
dans laquelle
R² et R³ représentent chacun, indépendamment l'un de l'autre, un groupe alkyle en C₁-C₆ ou cycloalkyle en C₅-C₇,
X représente l'oxygène ou le groupe NH et
Y représente l'hydrogène ou un groupe de formule 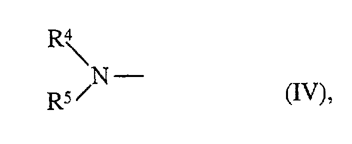 dans laquelle
R⁴ et R⁵ représentent chacun, indépendamment l'un de l'autre, un groupe alkyle en C₁-C₆ ou cycloalkyle en C₅-C₇,
sous réserve que dans le cas où X = NH, Y ne peut représenter qu'un groupe de formule (IV) et
dans les cas où X = oxygène et Y = un groupe de formule (IV), R⁴ et R⁵ peuvent également former ensemble
a) -(CHR⁶)ₙ- avec n = nombre entier allant de 2 à 4,
b) -CHR⁶-O-CHR⁶- ou
c) 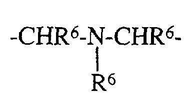
les symboles R⁶ représentant chacun, indépendamment les uns des autres, l'hydrogène, un groupe alkyle en C₁-C₆ ou cycloalkyle en C₅-C₇,
on opère en présence d'un diluant à des températures de réaction de 50 à 150°C, on met en oeuvre le catalyseur de formule (III) en quantité correspondant à 0,5 à 200 mol %, par rapport à la benzoisothiazolone de formule (II) mise en oeuvre, on met en oeuvre 1,2 à 10 mol de phosgène par mole de la benzoisothiazolone de formule (II) et après la fin de l'absorption du phosgène, on agite pendant encore quelque temps à la température de réaction ou à une autre température dans l'intervalle de 50 à 150°C, on élimine ensuite l'excès de phosgène puis le cas échéant le diluant à pression normale ou sous léger vide, et on isole le produit de réaction par distillation sous vide.

2. Procédé selon la revendication 1, **caractérisé en ce que**, dans les formules, l'halogène mentionné consiste en fluor, chlore ou brome,
un groupe alkyle en C₁-C₆ consiste en un groupe méthyle, éthyle, n-propyle, isopropyle, n-butyle, isobutyle, sec.-butyle, tert.-butyle, amyle ou hexyle,
un groupe alcoxy en C₁-C₆ consiste en un groupe méthoxy, éthoxy, n-propoxy, isopropoxy, n-butoxy, isobutoxy, sec.-butoxy, tert.-butoxy, amyloxy ou hexyloxy et
un groupe cycloalkyle en C₅-C₇ consiste en un groupe cyclopentyle, cyclohexyle ou cycloheptyle.

3. Procédé selon les revendications 1 et 2, **caractérisé en ce que** l'on met en oeuvre des benzoisothiazolones de formule (II) dans laquelle R¹ représente l'hydrogène ou le chlore.

4. Procédé selon les revendications 1 à 3, **caractérisé en ce que** l'on utilise des catalyseurs de formule (III) pour lesquels
a) X représente l'oxygène, Y l'hydrogène, et R² et R³, ayant des significations identiques, représentent chacun un groupe alkyle en C₁-C₄,
b) X représente l'oxygène ou NH, Y représente un groupe de formule (IV), R² à R⁵ ont des significations identiques et représentent chacun un groupe méthyle ou éthyle, ou bien
c) X représente l'oxygène, Y un groupe de formule (IV), R² et R³ ont des significations identiques et représentent chacun un groupe alkyle en C₁-C₄, et R⁴ et R⁵ forment ensemble un groupe -(CH₂)₂- ou -(CH₂)₃-.

5. Procédé selon les revendications 1 à 4, **caractérisé en ce que** l'on mélange le composant de départ, le catalyseur et le cas échéant le diluant et on injecte du phosgène à la température de réaction voulue.
